# EUROPEAN PATENT SPECIFICATION

(11) **EP 2 229 141 B1**
(45) Date of publication and mention of the grant of the patent: **23.08.2017**
(21) Application number: 08860087.9
(22) Date of filing: 10.12.2008
(51) Int. Cl.: A61K 8/60, A61Q 5/04, G01N 33/50

(54) **METHOD OF SELECTING AGENTS FOR MODIFYING THE SHAPE OF THE HAIR AND COSMETIC USE OF THE ACTIVE AGENTS**
VERFAHREN ZUR AUSWAHL VON MITTELN ZUR MODIFIZIERUNG DER FORM DES HAARES UND KOSMETISCHE VERWENDUNG VON WIRKSTOFFEN DAFÜR
PROCÉDÉ DE SÉLECTION D'AGENTS PERMETTANT DE MODIFIER LA FORME DU CHEVEU ET EMPLOI COSMÉTIQUE DES AGENTS ACTIFS

(30) Priority: 10.12.2007 FR 0759675; 19.12.2007 US 14910
(43) Date of publication of application: 22.09.2010
(73) Proprietor: L'Oréal, 75008 Paris (FR)
(72) Inventor: MALGOURIES, Sylvain, F-75017 Paris (FR); THIBAUT, Sébastien, F-92800 Puteaux (FR)
(74) Representative: Pillet, Anne-Helene
(86) International application number: PCT/EP2008/067240
(87) International publication number: WO 2009/074613

(56) References cited:
- WO-A-2006/040445
- US-A- 5 200 175
- US-A1- 2004 048 785
- US-A1- 2006 257 344
- US-B1- 6 495 147

## Description

The present invention relates to a cosmetic treatment process for keratin fibres and/or for their support, such as hairy skin, in particular for the hair and/or the scalp, with a view to modifying the shape of the keratin fibres in a long-lasting manner, more specifically for straightening them and smoothing them out.

It is known that two techniques are conventionally used for permanently reshaping the hair. They are based on cleavage of the disulphide bonds present in keratin (cystine):
- the first comprises, in a first stage, performing this opening of the disulphide bonds using a composition containing a reducing agent, and then, after having preferably rinsed the hair, reconstituting said disulphide bonds in a second stage, by applying to the hair, which has been placed under tension beforehand with rollers or the like or shaped or smoothed out by other means, an oxidizing composition also known as a fixer, so as to give the head of hair the desired shape. This technique equally makes it possible either to make the hair wavy or to straighten it, uncurl it or smooth it out.
- The second comprises performing a "lanthionization" operation using a composition containing a base belonging to the hydroxide family. This leads to a replacement of the disulphide bonds (-CH₂-S-S-CH₂-) with lanthionine bonds (-CH₂-S-CH₂-). This technique does not require a fixing reaction and is therefore carried out in a single stage.
These techniques give good results and the new shape imposed on the hair is long-lasting and withstands in particular the action of washing with water or with shampoos. However, they involve the use of substances of which the odour may be deemed unpleasant by the user and may be tricky to implement. In certain cases, these treatments may result in the hair becoming brittle.
There still therefore exists a need to find new methods which are suitable for the cosmetics field, which are not very aggressive for the hair and the scalp and which make it possible to modify the natural shape of said hair in a long-lasting manner.

Glycosaminoglycans (GAGs) are anionic polysaccharides grafted onto protein cores. GAGs group together chondroitin sulphates (CS), heparan sulphates (HS), keratan sulphates (KS) and dermatan sulphates (DS) according to their chemical structure.
Glycosylated proteins have for a long time been considered to be structural compounds of the extracellular matrix. Their involvement in many functions, such as cell communication, proliferation or differentiation, has only recently been demonstrated.

Unexpectedly, it has been found, in the context of the present invention, that in individuals with curly hair, the distribution of glycosaminoglycans (or GAGs) in the bulb of the hair follicle is asymmetrical.
It is therefore now possible to modify the shape of the keratin fibre, in particular newly synthesized by the hair follicle, by acting on the distribution of the GAGs, in particular by modulating the amount thereof at the level of the hair follicle. In fact, increasing the synthesis of GAGs in the follicle of the curly hair will have the effect of making the distribution thereof uniform and therefore of making the head hair or body hair taut and/or straightening it.
In the same way, agents which inhibit the synthesis of GAGs in follicles, by accentuating this distribution asymmetry, will increase the curliness or the curving of the head hair, of the body hair or of the eyelash.
The present invention describes the use of compounds which modulate the synthesis or degradation of GAGs, for modulating the shape of keratin fibres, in particular newly synthesized by the hair follicle.
The present invention also describes the use of compounds which stimulate the synthesis and/or secretion of PGs and/or of GAGs, and/or which decrease the degradation of GAGs, as an agent for modulating the shape of keratin fibres, in particular for straightening them or smoothing them out.
The present invention also describes the use of compounds which decrease or inhibit the synthesis and/or secretion of PGs and/or of GAGs, and/or which stimulate the degradation of GAGs, as an agent for modulating the shape of keratin fibres, in particular for increasing, promoting or maintaining the curly and/or frizzy appearance of keratin fibres, or promoting waviness, and/or increasing the curve of keratin fibres.
Thus, a subject of the invention is the cosmetic use of at least one active agent that can be selected by means of the method described hereafter, characterized in that the active agent is chosen from derivatives of iduronosyl anhydromannitol 6-sulphate (IMs), iduronosyl anhydromannitol, phenyl iduronide (PhI) or of 4-methylumbelliferyl iduronide, S-, N- and O-Glycosyl derivatives of the compound 2-acetamido-2-deoxy-D-glucose, or derivatives of 4-hydroxy-2-imino-1-methylpyrrolidine-5-carboxylic acid and of 2-imino-1-methylpyrrolidine-5-carboxylic acid, derivatives of aldonomonolactone or of alduronomonolactone, and acylated monosaccharides, for modifying the shape of keratin fibres straightening and/or smoothing out the hair.
A subject of the invention is also the cosmetic use of at least one active agent that can be selected by means of the method according to any one of Claims 1 to 3, characterized in that the active agent is chosen from 2-acetamido-3,4,6-tri-O-acetyl-1,5-anhydro-2-deoxy-D-glucitol, and uridine derivatives such as 5'-(6-O-[10-(2-naphthyl)-3,6,9-trioxadecanyl]-alpha-d-galactopyranosyl)diphosphate, for modifying the shape of keratin fibres.
The process and the use according to the invention are particularly suitable for modulating/modifying the shape of the newly synthesized fibre.
This constitutes an advantage of the invention according to which, rather than straightening a fibre which has grown curly, it is seen to that the fibre grows straight from its root onwards.
Compositions suitable for the implementation thereof are also included in the invention.

According to the invention, the term "keratin fibres" is intended to mean fibres of human or animal origin, such as the hair, body hairs, eyelashes, wool, angora, cashmere or fur. Although the invention is not limited to particular keratin fibres, reference will nevertheless more particularly be made to the hair and to the eyelashes.

Application WO 99/24009 proposes the use of D-xylose or of certain esters thereof for improving the functionality of epidermal cells, by stimulating the synthesis of PGs and of GAGs.
EP 531111 relates to compositions for promoting hair growth, containing a monosaccharide derivative which is a glycosaminoglycanase inhibitor.
EP0277428 relates to the use of inhibitors of proteoglycanase and glycosaminoglycanases for improving hair growth.
EP0398669 relates to sugar lactone glucuronide derivatives for improving hair growth.
EP0348184 relates to glycosaminoglycanase inhibitors of the aldonomonolactone, alduromonolactone and acetylated monosaccharide type for improving hair growth.
EP211610 and EP354595 describe the use of oligosaccharides, in particular of disaccharides comprising a uronic acid residue, for promoting hair growth.
WO 02/051828 describes novel C-glycoside derivatives and the use thereof, in particular for stimulating GAG synthesis. It is in particular proposed to incorporate them into an anti-hair-loss lotion or gel.
WO 2006/090307 reports the use of C-glycoside derivatives for improving the mechanical strength of keratin fibres, and in particular for preventing breaking of the hair.

However, to the applicant's knowledge, it has never been proposed to use compounds which stimulate the synthesis of GAGs and/or PGs, or which decrease the degradation of GAGs, for straightening and/or smoothing out the hair, body hair and/or the eyelashes. Conversely, it has never been proposed to use compounds which decrease or inhibit the synthesis and/or secretion of PGs and/or of GAGs, and/or which stimulate the degradation of GAGs, for making keratin fibres wavy or curling them. The terms "straightening", "smoothing out", "making wavy" or "curling" are herein intended to mean the modification of the shape of the fibre, and more especially during the production of the latter by the hair follicle.

In fact, it has now been demonstrated that the distribution of GAGs in the hair follicle is not uniform, and that the expression of some of them greatly decreases under the cells of the matrix, i.e. under the proliferative compartment of the follicle. This is in particular the case for perlecan and the heparan sulphate chains, which are greatly under-represented in the basal membrane. Similarly, nonsulphated and 4-sulphated chondroitins, D-type chondroitins and keratan sulphates are greatly decreased in the connective sheath. This decrease in expression could be correlated with the proliferative capacity of the matrix cells.
In addition, surprisingly, the inventors have demonstrated that the distribution of these glycosaminoglycans is asymmetrical, in the bulb of a curly hair, or in the follicle of a curved eyelash. This asymmetry concerns in particular the following compounds: perlecan, heparan sulphates, nonsulphated and 4-sulphated chondroitins, D-type chondroitins, and keratan sulphates. The decrease in GAG expression reported above is greater on the convex side of the curvature of the follicle.
The use of modulators of the synthesis and/or degradation of GAGs, in particular of an inhibitor of degradation, makes it possible, by making the synthesis of GAGs in the bulb of the hair, in particular of the curly hair, uniform, to even out their distribution. The use of activators of the synthesis and/or inhibitors of the degradation of GAGs according to the invention will thus lead to the hair becoming taut, and in the case of frizzy or curly hair, to the hair being smoothed out and/or straightened.
A new approach for modifying the shape of keratin fibres, in particular the hair or the eyelash, is thus found.
For this reason, a subject of the present invention is a method of selecting an active agent for modifying the shape of keratin fibres, characterized in that it comprises the following steps:
a) culturing mammalian cells,
b) adding the test substance to the mammalian cell culture,
c) after incubation, assaying the amount of glycosaminoglycans (GAGs) in the mammalian cells or in the mammalian cell culture medium,
d) comparing the amount of GAGs measured in step c with that measured starting from a control culture of mammalian cells, obtained under the same conditions, but in the absence of test substance,
e) selecting the substances for which the amount of GAGs is modified by at least a factor of 0.5 relative to the amount measured in the control culture.

The mammalian cells that can be used for implementing the method according to the invention may be chosen by those skilled in the art from skin cells and/or hair follicle cells, or else from immortalized lines such as HEK, CHO, HaCat, 3T3, etc. Fibroblasts or keratinocytes will in particular be used. Advantageously, the fibroblasts are chosen from the fibroblasts of the connective sheath of the hair, the fibroblasts of the dermal papilla and the fibroblasts of human dermis.
In particular, the method of selecting an active agent for modifying the shape of keratin fibres according to the invention will comprise the following steps:
a) culturing fibroblasts,
b) adding the test substance to the fibroblast culture,
c) after incubation, assaying the amount of glycosaminoglycans (GAGs) in the fibroblasts or in the fibroblast culture medium,
d) comparing the amount of GAGs measured in step c with that measured starting from a control culture of fibroblasts, obtained under the same conditions, but in the absence of test substance,
e) selecting the substances for which the amount of GAGs is modified by at least a factor of 0.5 relative to the amount measured in the control culture.

The steps for culturing the mammalian cells, in particular fibroblasts or keratinocytes, will be carried out by techniques and in media known to those skilled in the art.
In step c, the test substance will be left in contact with the fibroblasts or the keratinocytes for a period of time sufficient for the GAG metabolism to be influenced, where appropriate, by the presence of said substance. This period of time is between a few hours and a few days, especially from 6 h to 15 days, in particular from 1 to 6 days.
Similarly, the assaying of the GAGs will be carried out by methods known to those skilled in the art, such as the use of cationic dyes, for instance 1,9-dimethylmethylene blue (DMMB) (Barbosa, I. et al., Improved and simple micro assay for sulfated glycosaminoglycans quantification in biological extracts and its use in skin and muscle tissue studies, Glycobiology, 2003, 13, 647-653) or O-safranin (Lammi, M. et al., Densitometric assay of nanogram quantities of proteoglycans precipitated on nitrocellulose membrane with Safranin O, Anal Biochem, 1988, 168, 352-357), or by assaying uronic acid via the carbazole reaction (Dische, Z. et al., Two modifications of the carbazole reaction of hexuronic acids for the differentiation of polyuronides, Anal Biochem, 1967, 21, 125-130).
Substances capable of modulating the GAG concentration in the hair follicle, and therefore of modifying the shape of the hair, body hair and/or eyelashes in a long-lasting manner, will thus be selected. After the cells have been cultured in the presence of the compounds or mixtures of compounds selected, the GAG concentration may in fact increase by 1.5 times, or, on the contrary, be less than or equal to 0.5 times the concentration measured in the control culture.
According to one of the embodiments of the method of the invention, in step e, the substances for which the amount of GAGs is at least equal to 1.5 times the amount measured in the control culture are selected.
According to another embodiment of the method according to the invention, in step e, the substances for which the amount of GAGs is decreased at least by half relative to the amount measured in the control culture are selected.
Thus, the present invention describes the cosmetic use, in a composition containing a physiologically acceptable medium, as an agent for modifying the shape of keratin fibres, of at least one modulator of the synthesis and/or secretion and/or degradation of GAGs, as described hereafter, this active agent being capable of being selected by the method defined above, the modulator or the composition containing it being for use in modifying the shape of keratin fibres.

The cosmetic use according to the invention is more particularly suitable for keratin fibres which are human hair, human eyelashes and/or human body hair.
More particularly, the agent which stimulates the synthesis and/or secretion of GAGs and of PGs and/or the agent which decreases the degradation of GAGs, or the composition containing it, is for use in smoothing out and/or straightening the hair and/or the eyelashes.
The term "straightening" encompasses, according to the invention, the straightening, smoothing out or uncurling of Caucasian, Asian, North African or African hair.

According to another embodiment, a subject of the invention is the cosmetic use, in a composition containing a physiologically acceptable medium, as an agent for modifying the shape of keratin fibres, of at least one active agent chosen from agents which decrease or inhibit the synthesis and/or secretion of PGs and/or GAGs, and/or which stimulate the degradation of GAGs as described hereafter.
The hair shaping according to the invention encompasses the curling, permanent-waving and setting of hair, in particular Caucasian, Asian, North African, Afro-American or African hair.

A subject of the invention is also the cosmetic use of at least one activator of the synthesis and/or secretion of GAGs and/or of an inhibitor of the degradation of GAGs as defined hereafter, as an agent for straightening and/or smoothing out the hair.

The agents that are of use for implementing the invention, for modifying the shape of keratin fibres, are modulators of the synthesis and/or secretion and/or degradation of GAGs, and are chosen from compounds as described hereafter.

According to the present invention, use may be made of agents which inhibit the synthetic and elongation enzymes:
2-acetamido-3,4,6-tri-O-acetyl-1,5-anhydro-2-deoxy-D-glucitol;
Uridine derivatives, such as 5'-(6-O-[10-(2-naphthyl)-3,6,9-trioxadecanyl]-alpha-d-galactopyranosyl)diphosphate (beta-1,4-galactosyltransferase inhibitor).

Other compounds that are useful according to the invention are inhibitors of the degradation enzymes:
Derivatives of iduronosyl anhydromannitol 6-sulphate (IMs), iduronosyl anhydromannitol, phenyl iduronide (PhI) or of 4-methylumbelliferyl iduronide (alpha-L-iduronidase inhibitors).
S-, N- and O-glycosyl derivatives of the compound 2-acetamido-2-deoxy-D-glucose, or derivatives of 4-hydroxy-2-imino-1-methylpyrrolidine-5-carboxylic acid and 2-imino-1-methylpyrrolidine-5-carboxylic acid from streptomyces (alpha-N-acetylglucosaminidase inhibitors).
Derivatives of aldonomonolactone or of alduronomonolactone, and acylated monosaccharides (glycosaminoglycanase inhibitors), and more generally sugar lactone derivatives. Such compounds are mentioned in applications EP0384184, EP0277428, EP0398669 and EP 531111.
These compounds or the compositions containing them are preferably for straightening and/or smoothing out the hair.

Advantageously, the modulator of the synthesis and/or secretion and/or degradation of GAGs according to the invention is applied in the form of a composition containing a physiologically acceptable medium, i.e. a medium compatible with the skin and/or the mucous membranes. The compositions according to the invention are in particular cosmetic or dermatological compositions; they are in particular a composition suitable for caring for, conditioning, making up, removing makeup from, protecting, cleansing or washing keratin fibres.
For oral administration, the composition of the invention may be in any of the suitable forms, particularly in the form of an oral solution, a tablet, a gel capsule, a capsule or else a nutritional food or a nutritional supplement.
Said composition also comprises at least one appropriate excipient suitable for oral administration.

In these compositions, the concentration of the modulator of the synthesis and/or secretion and/or degradation of GAGs may be between 10⁻³ and 30% by weight; it is in general greater than or equal to 0.01% by weight, relative to the total weight of the composition, in particular greater than or equal to 0.1%; the effective amounts will be adjusted by those skilled in the art according to the desired result and the modulators of the synthesis and/or secretion and/or degradation of GAGs used, but are generally less than or equal to 30%, in particular less than or equal to 15% by weight, or even less than or equal to 10%.
The compositions may be in any of the forms suitable for caring for the hair and/or the scalp, in particular in the form of a haircare lotion, for example for daily or twice-weekly application, a shampoo or a hair conditioner, in particular for weekly or twice-weekly application, a liquid or solid soap for cleansing the scalp, for daily application, a product for shaping the hairstyle (lacquer, hairstyling gel), a treatment mask, or a foaming gel or cream for cleansing the hair.

A composition suitable for implementing the invention may be in the form of an aqueous, alcoholic or aqueous-alcoholic solution or suspension, an oily suspension, an emulsion of more or less fluid and in particular liquid, semi-liquid or solid consistency, obtained by dispersion of a fatty phase in an aqueous phase (O/W) or vice versa (W/O), or a multiple emulsion, an aqueous, aqueous-alcoholic or oily gel, a loose or compacted powder to be used as it is or to be incorporated into a physiologically acceptable medium, microcapsules or microparticles, or ionic or nonionic dispersions.
Advantageously, the modulator of the synthesis and/or secretion and/or degradation of GAGs is applied in the form of a formulation which promotes the penetration of the agent into the hair follicle.
The compositions may, in certain cases, contain penetration accelerators, in particular chosen from noncyclic monoalcohols and dialcohols, ethyl acetate, butyl acetate, isopropyl myristate, fatty acids, phospholipids, terpenes, azone and derivatives, propylene glycol and glycolic derivatives, cyclodextrins, octyl salicylate, cyclopentadecanolid, polysorbates, and polyvinylpyrrolidone and derivatives, this list not being limiting.

The modulator(s) of the synthesis and/or secretion and/or degradation of GAGs may in particular be used in a formulation which includes individualized porous particles characterized in that they have a volume-average diameter of less than or equal to 10 µm and a specific surface area of greater than or equal to 1 m²/g, as described in French patent 2856594.

According to one embodiment, the compositions also contain a surfactant of nonionic, anionic, cationic or amphoteric type, and among the latter, mention may be made of alkyl sulphates, alkyl benzenesulphates, alkyl ether sulphates, alkyl sulphonates, quaternary ammonium salts, alkylbetains, oxyethylenated alkylphenols, fatty acid alkanolamides, oxyethylenated fatty acid esters, and also other nonionic surfactants of the hydroxypropyl ether type.

When the compositions contain at least one surfactant, the latter is generally present at a maximum concentration of 30% by weight, and preferably between 0.5% and 10% by weight, relative to the total weight of the composition.

With the aim of improving the cosmetic properties of the hair or alternatively of reducing or preventing the degradation thereof, the composition may also contain a treatment agent of cationic, anionic, nonionic or amphoteric nature.
Among the treatment agents which are particularly preferred, mention may especially be made of those described in French patents No. 2.598.613 and No. 2.470.596. As treatment agents, use may also be made of linear or cyclic, volatile or non-volatile silicones and mixtures thereof, polydimethylsiloxanes, quaternized polyorganosiloxanes such as those described in French patent application No. 2.535.730, polyorganosiloxanes comprising aminoalkyl groups modified with alkoxycarbonylalkyl groups, such as those described in US patent No. 4.749.732, polyorganosiloxanes such as the polydimethylsiloxane-polyoxyalkyl copolymer of the Dimethicone Copolyol type, a polydimethylsiloxane containing stearoxy (stearoxydimethicone) end groups, a polydimethylsiloxane- dialkylammonium acetate copolymer or a polydimethylsiloxane-polyalkylbetain copolymer described in British patent No. 2.197.352, polysiloxanes organomodified with mercapto or mercaptoalkyl groups, such as those described in French patent No. 1.530.369 and in European patent application No. 295.780, and also silanes such as stearoxytrimethylsilane.

The compositions according to the invention may also contain other treatment ingredients such as cationic polymers, for instance those used in the compositions of French patents No. 79.32078 (2.472.382) and 80.26421 (2.495.931), or else cationic polymers of the ionene type, such as those used in the compositions of Luxembourg patent No. 83703, basic amino acids (such as lysine or arginine) or acidic amino acids (such as glutamic acid or aspartic acid), peptides and derivatives thereof, protein hydrolysates, waxes, swelling agents and penetrating agents such as the SiO₂/PDMS (polydimethylsiloxane) mixture, dimethylisosorbitol, urea and its derivatives, pyrrolidone, N-alkylpyrrolidones, thiamorpholinone, alkylene glycol alkyl ethers or dialkylene glycol alkyl ethers, for instance propylene glycol monomethyl ether, dipropylene glycol monomethyl ether, ethylene glycol monoethyl ether and diethylene glycol monoethyl ether, 2-imidazolidinone, and also other compounds such as fatty alcohols, lanolin derivatives, active agents such as panthothenic acid, agents for preventing hair loss, antidandruff agents, thickeners, suspension agents, sequestering or complexing agents, opacifiers, sunscreens and also fragrances and preservatives.

The compositions according to the invention are in particular in the form of a thickened cream so as to keep the hair as stiff as possible. These creams are prepared in the form of "heavy" emulsions, for example based on glyceryl stearate, on glycol stearate, on self-emulsifiable waxes or on fatty alcohols.

Use may also be made of liquids or gels containing thickeners, such as carboxyvinyl polymers or copolymers which "stick" the hairs together and keep them in the smooth position during the leave-in time.

The compositions according to the invention may also contain at least one adjuvant chosen from silicones in soluble, dispersed or microdispersed form, nonionic, anionic, cationic and amphoteric surfactants, ceramides, glycoceramides and pseudoceramides, vitamins and provitamins, including panthenol, plant, animal, mineral and synthetic oils, waxes other than ceramides, glycoceramides and pseudoceramides, silicone-based or nonsilicone-based, water-soluble and fat-soluble sunscreens, pearlescent agents and opacifiers, sequestering agents, plasticizers, solubilizing agents, acidifying agents, mineral and organic thickeners, antioxidants, hydroxy acids, penetration agents, fragrances and preservatives. Among the solubilizing agents, mention may, for example, be made of lower alcohols such as, for example, ethanol, propanol or isopropanol.

The compositions suitable for the invention contain at least one, and in particular at least two, modulators of the synthesis and/or secretion and/or degradation of GAGs. However, an active agent which increases the concentration of GAGs will preferably not be combined with an active agent which decreases this same concentration.

According to one particular embodiment, the compositions also contain at least one other active agent that is beneficial to the health and vigour of the hair and of body hair, in particular at least one active agent which promotes hair regrowth and/or which limits hair loss. It may in particular be an inhibitor of the 15-hydroxyprostaglandin dehydrogenase enzyme, such as those described in application WO 2004/073594, or esters of vitamin F and of glucose described in application EP 1371658.

The invention also describes a cosmetic treatment process for keratin fibres in a mammal, with a view to modifying their shape, characterized in that at least one modulator of the synthesis and/or secretion and/or degradation of GAGs, or a composition containing it, as defined above, is administered to the mammal. The mammal is preferably a human being.

The modulator of the synthesis and/or secretion and/or degradation of GAGs, or the composition containing it, may be applied to the keratin fibres or to their support, in particular to the hair and/or the scalp.

The process may, for example, be carried out by applying the modulator(s) of the synthesis and/or secretion and/or degradation of GAGs or the compositions containing them, as defined above, to the hair and/or the scalp, daily or twice a day, for a period preferably of at least one week, and in particular from 2 to 6 weeks. The modulator of the synthesis and/or secretion and/or degradation of GAGs or the composition may be rinsed off after a sufficient contact time, in particular from 5 to 60 minutes, or else may be applied, for example, in the form of a leave-in lotion or nourishing cream and remain in contact with the hair and/or the scalp until the next shampooing operation. The process makes it possible to obtain a long-lasting modification of the shape of the hair, since it acts on the structure of the hair as soon as the latter is formed. It will be particularly suitable for individuals who have naturally curly or frizzy hair; in one particular embodiment, the hair will be placed under tension or shaped or smoothed out by appropriate means.

According to another embodiment, the modulator of the synthesis and/or secretion and/or degradation of GAGs or the composition containing it will be administered to the mammal orally.

Other characteristics and advantages of the invention will emerge on reading the examples which follow.

### Example 1:

### Demonstration of the asymmetrical distribution of GAGs on curly hair

### 1. a) Dissection of hair follicles

Hair follicles originating from human scalp biopsies are dissected according to the method described in patent FR2736721A1 of 17/01/97; US5712169A of 28/01/98.
The isolated follicles are conserved in Tissue-Tek OCT compound (Miles, Naperville, IL, USA) and frozen in a dry ice/ethanol mixture.

### 1. b) Preparation of cryosections

Longitudinal 7 µm-thick sections of isolated hair follicles or scalp biopsies are cut using a CM3050 cryostat (Leica, Rueil Malmaison, France), in which the object temperature is maintained at - 40°C and the chamber temperature is maintained at -35°C. The sections are fixed in air and then conserved for 24 hours at +4°C.

### 1. c) Immunolabelling

The sections are fixed with acetone at -20°C for 10 minutes and then rinsed in a phosphate-buffered saline (PBS) (Sigma, Saint-Quentin Fallavier, France). The endogenous peroxidases are saturated using 0.1% hydrogen peroxide (Sigma, Saint-Quentin Fallavier, France).

### List of the antibodies used:

| Primary antibody | Ig class | Specificity | | Dilution | Supplier |
|---|---|---|---|---|---|
| F58-10E4 | Mouse IgM | heparan sulphate | | 1/50 | Seikagaku corporation, Tokyo, Japan |
| HepSS-1 | Mouse IgM | heparan sulphate | | 1/30 | Seikagaku corporation, Tokyo, Japan |
| | | | | 1/100 | |
| 1-B-5 | Mouse IgG1 | Unsulphated chondroitin or Dermatan (chABC) | | 1/50 | Seikagaku corporation, Tokyo, Japan |
| 2-B-6 | Mouse IgG1 | 4-sulphated chondroitin (chABC) | | 1/20 | ICN Biomedicals |
| BE-123 | Mouse IgG1 | 4-sulphated chondroitin (chABC) | | 1/20 | MP Biomedicals, Aurora, OH, USA |
| 3-B-3/C1 | Mouse IgM | 6-sulphated chondroitin (chABC) | | 1/20 | ICN Biomedicals, Costa Mesa, CA, USA |
| MK-302 | Mouse IgG1 | 6-sulphated chondroitin (chABC) | | 1/20 | MP Biomedicals, Aurora, OH, USA |
| MO-225 | Mouse IgM | Chondroitin-sulphate type D | | 1/50 | Seikagaku corporation, Tokyo, Japan |
| 5-D-4 | Mouse IgG1 | Keratan-sulphate | | 1/50 | Seikagaku corporation, Tokyo, Japan |
| 7B5 | Mouse IgG1 | Perlecan | | 1/100 | Zymed, South San Francisco, CA, USA |
| | | | | | |

| Secondary antibody | Conjugate | Specificity | Dilution | Host | Supplier |
|---|---|---|---|---|---|
| 705-065-147 | Biotin | Goat | 1/400 | Donkey | Jackson, Baltimore, PE, USA |
| E0433 | Biotin | Mouse IgG | 1/400 | Goat | DAKO, Glostrup, Denmark |
| E432 | Biotin | Rabbit | 1/400 | Goat | DAKO, Glostrup, Denmark |
| A31570 | Alexafluor 555 | Mouse IgG | 1/100 | Donkey | Molecular Probes, Eugene, OR, USA |
| A11055 | Alexafluor 488 | Goat | 1/100 | Donkey | Molecular Probes, Eugene, OR, USA |

The labelling is then analysed with a Zeiss Axioscope microscope (Carl Zeiss, Oberkochen, Germany).

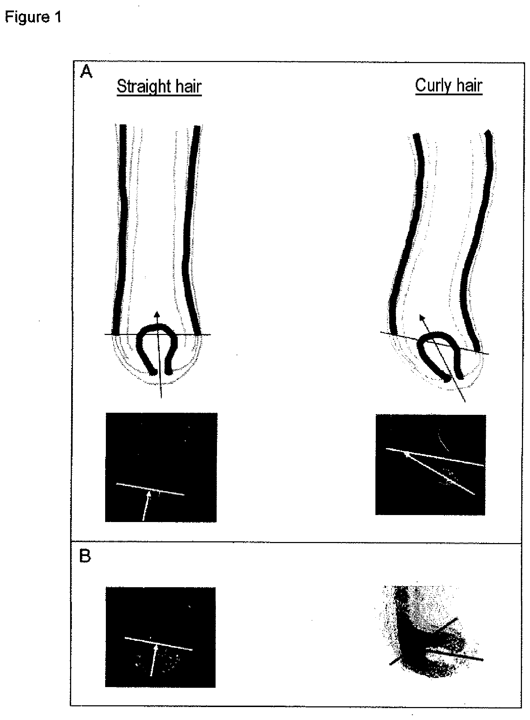

The results are represented in Figure 1.
An asymmetrical distribution of the heparan sulphate chains is observed in the bulb of a frizzy hair, compared with a straight hair. Figure 1B shows the asymmetrical proliferation which results therefrom.

### Example 2 (out of the invention):

### Increase in GAG synthesis with a xyloside derivative

### Fibroblast culture:

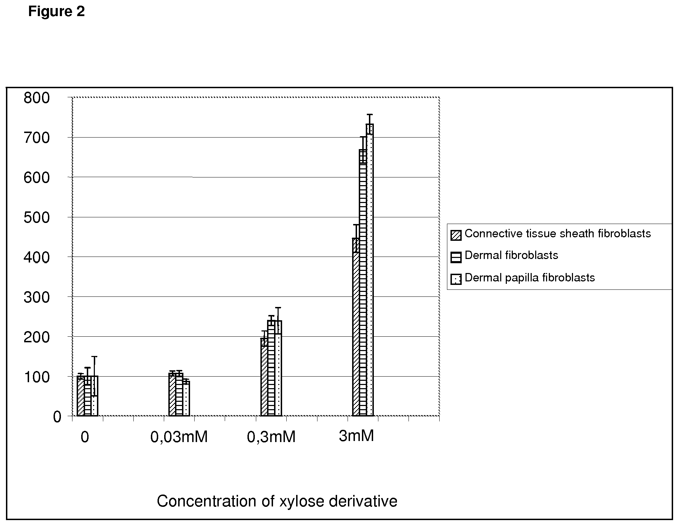

The fibroblasts are cultured in DMEM medium, 10% foetal calf serum (Gibco, Invitrogen), 1 mM MEM pyruvate (Gibco, Invitrogen) and 25 units/ml of penicillin-streptomycin (Gibco, Invitrogen). The dermal papilla fibroblast (P6), connective sheath fibroblast (P6) and dermal fibroblast are then counted using a Coulter Counter Z2 (Beckman Coulter inc., Fullerton, USA) and seeded at 2 × 10⁵ cells per well in a 12-well plate, with the wells containing 1.5 ml of medium alone, which is the "control" condition, or being treated with 3mM of 1,5-anhydro-6,8-dideoxy-L-glucooctitol. After 7 days, the supernatant is recovered and stored at -80°C until the GAGs are assayed. The cells are lysed with 130 µl of 100mM tris-HCl buffer containing 1% triton, 10% glycerol and 2% anti-proteases, at pH 7.2, by carrying out three freezing at -80°C/thawing at 37°C cycles, mechanical scraping of the cells, and then a sonication step.
The proteins present in the lysate are assayed using the Dc Protein assay kit (Biorad).
The amount of glycosaminoglycans is measured in the supernatant and the lysate.
Assaying of sulphated glycosaminoglycans:
The glycosaminoglycans are assayed using the Blyscan glycosaminoglycan isolation & concentration kit (Biocolor, Newtownabbey, Northern Ireland) and the Blyscan sulphated glycosaminoglycan assay kit. The assay is performed on 700 µl and 400 µl of supernatant, 50 µl of lysate and a range of 0, 0.5, 1, 2, 3, 4 and 5 µg of supplied chondroitin-4-sulphate. Each sample to be measured is mixed with 700 µl of the cetylpyridinium chloride solution in a 1.5 ml centrifuge tube, and then incubated on a rotary mixer thermostated at 37°C, at 250 rpm, for 2 hours. The tubes are centrifuged for 15 min at 10 000 rpm in an Eppendorf 5415C centrifuge. The pellet is mixed with 1 ml of 95% ethanol and incubated in a rotary mixer thermostated at 37°C, at 250 rpm, for 5 min. The tube is again centrifuged for 15 min at 10 000 rpm. The ethanol is removed and the tube is dried in the open air for a few minutes. 1 ml of Blyscan dye reagent is added directly to the pellet and vortexed for 30 min at ambient temperature before being centrifuged at 14 000 rpm for 15min. 1 ml of Blyscan dissociation reagent is added to the pellet. The whole is vortexed for 15 min until complete dissolution. The absorbance of the solution at 656 nm is measured using a polystyrene semi-microcuvette on a Biomate 5 spectrometer (Thermo). The amount of glycosaminoglycan present is then measured.
The results are represented in Figure 2.
An increase in GAG synthesis by the fibroblasts, proportional to the concentration of 1,5-anhydro-6,8-dideoxy-L-glucooctitol, is observed. The addition of this active agent increases the amount of glycosaminoglycans secreted by up to 7 times.

### Example 3: Hair-straightening composition

| | |
|---|---|
| Ammonium polyacryloyldimethyltaurate | 0.5 g |
| Porous particles of Nylon-12* | 4.7 g |
| 5-n-octanoylsalicylic acid | 0.3 g |
| Poloxamer 338 | 0.25 g |
| Iduronosyl anhydromannitol | 1g |
| Demineralized water | 93.25 g |

| | |
|---|---|
| * The porous particles of Nylon-12 are sold under the name "Orgasol 2002 UD Nat cos" by the company Atofina. | |

## Claims

1. Method of selecting an active agent for modifying the shape of keratin fibres, **characterized in that** it comprises the following steps:
a) culturing mammalian cells,
b) adding the test substance to the mammalian cell culture,
c) after incubation, assaying the amount of glycosaminoglycans (GAGs) in the mammalian cells or in the mammalian cell culture medium,
d) comparing the amount of GAGs measured in step c with that measured starting from a control culture of mammalian cells, obtained under the same conditions, but in the absence of test substance,
e) selecting the substances for which the amount of GAGs is modified by at least a factor of 0.5 relative to the amount measured in the control culture.

2. Selection method according to Claim 1, **characterized in that** the mammalian cells are fibroblasts or keratinocytes chosen from fibroblasts of the connective sheath of the hair, fibroblasts of the dermal papilla, fibroblasts of human dermis, skin keratinocytes, and keratinocytes of the outer sheath or of the epithelial matrix of the hair follicle.

3. Selection method according to at least one of the preceding claims, **characterized in that**, in step e, the substances for which the amount of GAGs is at least equal to 1.5 times the amount measured in the control culture are selected.

4. Selection method according to at least one of Claims 1 and 2, **characterized in that**, in step e, the substances for which the amount of GAGs is decreased at least by half relative to the amount measured in the control culture are selected.

5. Cosmetic useof at least one active agent that can be selected by means of the method according to any one of Claims 1 to 3, **characterized in that** the active agent is chosen from derivatives of iduronosyl anhydromannitol 6-sulphate (IMs), iduronosyl anhydromannitol, phenyl iduronide (PhI) or of 4-methylumbelliferyl iduronide, S-, N- and O-Glycosyl derivatives of the compound 2-acetamido-2-deoxy-D-glucose, or derivatives of 4-hydroxy-2-imino-1-methylpyrrolidine-5-carboxylic acid and of 2-imino-1-methylpyrrolidine-5-carboxylic acid, derivatives of aldonomonolactone or of alduronomonolactone, and acylated monosaccharides, for modifying the shape of keratin fibres straightening and/or smoothing out the hair.

6. Cosmetic useof at least one active agent that can be selected by means of the method according to any one of Claims 1 to 3, **characterized in that** the active agent is chosen from 2-acetamido-3,4,6-tri-O-acetyl-1,5-anhydro-2-deoxy-D-glucitol, and uridine derivatives such as 5'-(6-O-[10-(2-naphthyl)-3,6,9-trioxadecanyl]-alpha-d-galactopyranosyl)diphosphate, for modifying the shape of keratin fibres

7. Cosmetic use according to at least one of Claims 5 or 6, **characterized in that** the keratin fibres are chosen from human hair, body hair and/or eyelashes.

8. Use according to at least one of Claims 5 to 7, **characterized in that** the composition containing the agent for modifying the shape of keratin fibres is a composition for external topical administration.

9. Use according to the preceding claim, **characterized in that** the composition containing a physiologically acceptable medium is suitable for caring for, conditioning, making up, removing makeup from, protecting, cleansing or washing keratin fibres.

10. Use according to at least one of Claims 5 to 9, **characterized in that** the agent for modifying the shape of keratin fibres is in a composition which promotes its penetration into the hair follicle.

11. Use according to at least one of Claims 5 to 10, **characterized in that** the composition also contains at least one adjuvant chosen from silicones in soluble, dispersed or microdispersed form, nonionic, anionic, cationic and amphoteric surfactants, ceramides, glycoceramides and pseudoceramides, vitamins and provitamins, including panthenol, plant, animal, mineral and synthetic oils, waxes other than the ceramides, glycoceramides and pseudoceramides, silicone-based or nonsilicone-based, water-soluble and fat-soluble sunscreens, pearlescent agents and opacifiers, sequestering agents, plasticizers, solubilizing agents, acidifying agents, mineral and organic thickeners, antioxidants, hydroxy acids, penetration agents, fragrances and preservatives.

12. Use according to at least one of Claims 5 to 11, **characterized in that** the composition is a cosmetic composition.

## Patentansprüche

1. Verfahren zum Auswählen eines Wirkstoffes zum Modifizieren der Form von Keratinfasern, **dadurch gekennzeichnet, dass** es die folgenden Schritte umfasst:
a) Kultivieren von Säugetierzellen,
b) Zugeben der Versuchssubstanz zu der Säugetierzellkultur,
c) nach Inkubation, Bestimmten der Menge von Glykosaminoglykanen (GAGs) in den Säugetierzellen oder in dem Säugetierzellkulturmedium,
d) Vergleichen der in Schritt c gemessenen Menge von GAGs mit jener, die beginnend mit einer Kontrollkultur von Säugetierzellen, erhalten unter denselben Bedingungen aber in der Abwesenheit der Versuchssubstanz, gemessen wurde,
e) Auszählen der Substanzen, für welche die Menge von GAGs um mindestens einen Faktor 0,5 bezogen auf die in der Kontrollkultur gemessenen Menge modifiziert ist.

2. Auswahlverfahren nach Anspruch 1, **dadurch gekennzeichnet, dass** die Säugetierzellen Fibroblasten oder Keratinozyten sind, ausgewählt aus Fibroblasten der Bindegewebsscheide der Haare, Fibroblasten der Papilla dermis, Fibroblasten der menschlichen Dermis, Hautkeratinozyten, und Keratinozyten der äußeren Wurzelscheide oder der epithelialen Matrix des

3. Auswahlverfahren nach mindestens einem der vorhergehenden Ansprüche, **dadurch gekennzeichnet, dass** in Schritt e die Substanzen ausgewählt werden, für welche die Menge von GAGs mindestens dem 1,5-fachen der in der Kontrollkultur gemessenen Menge entspricht.

4. Auswahlverfahren nach mindestens einem der Ansprüche 1 und 2, **dadurch gekennzeichnet, dass** in Schritt e die Substanzen ausgewählt werden, für welche die Menge von GAGs bezogen auf die in der Kontrollkultur gemessenen Menge um mindestens die Hälfte verringert ist.

5. Kosmetische Verwendung von mindestens einem Wirkstoff, der mittels des Verfahrens nach einem der Ansprüche 1 bis 3 ausgewählt werden kann, **dadurch gekennzeichnet, dass** der Wirkstoff aus Derivaten von Iduronosylanhydromannitol-6-sulfat (IMs), Iduronosylanhydromannitol, Phenyliduronid (PhI) oder von 4-Methylumbelliferyliduronid, S-, N- und O-Glykosylderivaten der Verbindung 2-Acetamido-2-desoxy-D-Glucose, oder Derivaten von 4-Hydroxy-2-imino-1-methylpyrrolidin-5-carboxylsäure und von 2-Imino-1-methylpyrrolidin-5-carboxylsäure, Derivaten von Aldonomonolacton oder von Alduronomonolacton, und acylierten Monosacchariden ausgewählt ist, zum Modifizieren der Form von Keratinfasern, wobei das Haar gerade gebogen und/oder geglättet wird.

6. Kosmetische Verwendung von mindestens einem Wirkstoff, der mittels des Verfahrens nach einem der Ansprüche 1 bis 3 ausgewählt werden kann, **dadurch gekennzeichnet, dass** der Wirkstoff aus 2-Acetamido-3,4,6-tri-O-acetyl-1,5-anhydro-2-desoxy-D-glucitol, und Uridinderivaten wie 5'-(6-0-[10-(2-naphthyl)-3,6,9-trioxadecanyl]-alpha-d-galactopyranosyl)diphosphat ausgewählt ist, zum Modifizieren der Form von Keratinfasern.

7. Kosmetische Verwendung nach mindestens einem von Anspruch 5 oder 6, **dadurch gekennzeichnet, dass** die Keratinfasern aus menschlichem Haar, Körperhaar und/oder Wimpern ausgewählt sind.

8. Verwendung nach mindestens einem der Ansprüche 5 bis 7, **dadurch gekennzeichnet, dass** die Zusammensetzung, die das Mittel zum Modifizieren der Form von Keratinfasern enthält, eine Zusammensetzung zur äußerlichen topischen Anwendung ist.

9. Verwendung nach dem vorhergehenden Anspruch, **dadurch gekennzeichnet, dass** die Zusammensetzung, die ein physiologisch annehmbares Medium enthält, zur Pflege, zur Verbesserung, zum Make-up, zur Entfernung von Make-up aus, zum Schutz, zur Reinigung oder zum Waschen von Keratinfasern geeignet ist.

10. Verwendung nach mindestens einem der Ansprüche 5 bis 9, **dadurch gekennzeichnet, dass** das Mittel zum Modifizieren der Form von Keratinfasern in einer Zusammensetzung vorliegt, die seine Penetration in den Haarfollikel fördert.

11. Verwendung nach mindestens einem der Ansprüche 5 bis 10, **dadurch gekennzeichnet, dass** die Zusammensetzung auch mindestens einen Hilfsstoff enthält, der aus Silikonen in löslicher, dispergierter oder mikrodispergierter Form, nichtionischen, anionischen, kationischen und amphoteren oberflächenaktiven Substanzen, Ceramiden, Glykoceramiden und Pseudoceramiden, Vitaminen und Provitaminen, einschließlich Panthenol, Pflanzen-, Mineral- und synthetischen Ölen, Wachsen außer den Ceramiden, Glykoceramiden und Pseudoceramiden, silikonbasierten oder nicht silikonbasierten, wasserlöslichen und fettlöslichen Sonnenschutzmitteln, Perlglanzmitteln und Trübungsmitteln, Komplexbildnern, Weichmachern, Lösungsvermittlern, Säuerüngsmitteln, mineralischen und organischen Verdickungsmitteln, Antioxidantien, Hydroxysäuren, Penetrationsmitteln, Duftstoffen und Konservierungsmitteln ausgewählt ist.

12. Verwendung nach mindestens einem der Ansprüche 5 bis 11, **dadurch gekennzeichnet, dass** die Zusammensetzung eine kosmetische Zusammensetzung ist.

## Revendications

1. Méthode de sélection d'un agent actif destiné à modifier la forme de fibres kératiniques, **caractérisée en ce qu'**elle comprend les étapes suivantes :
a) la culture de cellules mammaliennes,
b) l'addition de la substance à tester à la culture de cellules mammaliennes,
c) après incubation, le dosage de la quantité de glycosaminoglycanes (GAG) dans les cellules mammaliennes ou dans le milieu de culture de cellules mammaliennes,
d) la comparaison de la quantité de GAG mesurée dans l'étape « c » avec celle mesurée en partant d'une culture témoin de cellules mammaliennes, obtenue dans les mêmes conditions, mais en l'absence de substance à tester,
e) la sélection des substances pour lesquelles la quantité de GAG est modifiée d'au moins un facteur de 0,5 par rapport à la quantité mesurée dans la culture témoin.

2. Méthode de sélection selon la revendication 1, **caractérisée en ce que** les cellules mammaliennes sont des fibroblastes ou des keratinocytes choisis parmi les fibroblastes de la gaine conjonctive du cheveu, les fibroblastes des papilles dermiques, les fibroblastes du derme humain, les kératinocytes cutanés, et les kératinocytes de la gaine externe ou de la matrice épithéliale du follicule pileux.

3. Méthode de sélection selon au moins l'une des revendications précédentes, **caractérisée en ce que**, dans l'étape « e », les substances pour lesquelles la quantité de GAG est au moins égale à 1,5 fois la quantité mesurée dans la culture témoin, sont sélectionnées.

4. Méthode de sélection selon au moins l'une des revendications 1 et 2, **caractérisée en ce que**, dans l'étape « e », les substances pour lesquelles la quantité de GAG est réduite au moins de moitié par rapport à la quantité mesurée dans la culture témoin, sont sélectionnées.

5. Utilisation cosmétique d'au moins un agent actif pouvant être sélectionné à l'aide de la méthode selon l'une quelconque des revendications 1 à 3, **caractérisée en ce que** l'agent actif est choisi parmi les dérivés de l'anhydromannitol 5-sulfate d'iduronosyle (IM), l'anhydromannitol d'iduronosyle, l'iduronide de phényle (PhI), ou l'iduronide de 4-méthylumbelliféryle, les dérivés de S-, N- et O-glycosyle du composé de 2-acétamido-2-désoxy-D-glucose, ou les dérivés d'acide 4-hydroxy-2-imino-1-méthylpyrrolidine-5-carboxylique et d'acide 2-imino-1-méthylpyrrolidine-5-carboxylique, les dérivés d'aldonomonolactone ou d'alduronomonolactone, et les monosaccharides acylés, afin de modifier la forme des fibres kératiniques, défrisant et/ou laissant les cheveux.

6. Utilisation cosmétique d'au moins un agent actif pouvant être sélectionné à l'aide de la méthode selon l'une quelconque des revendications 1 à 3, **caractérisée en ce que** l'agent actif est choisi parmi le 2-acétamido-3,4,6-tri-O-acétyl-1,5-anhydro-2-désoxy-D-glucitol, et les dérivés d'uridine tels que le 5'-(6-O-[10-(2-naphtyl)-3,6,9-trioxadécanyl]-alpha-D-galacto-pyranosyl)diphosphate, afin de modifier la forme des fibres kératiniques.

7. Utilisation cosmétique selon au moins l'une des revendications 5 ou 6, **caractérisée en ce que** les fibres kératiniques sont choisies parmi les cheveux humains, le système pileux et/ou les cils.

8. Utilisation selon au moins l'une des revendications 5 à 7, **caractérisée en ce que** la composition contenant l'agent destiné à modifier la forme de fibres kératiniques est une composition pour une administration topique externe.

9. Utilisation selon la revendication précédente, **caractérisée en ce que** la composition contenant un milieu physiologiquement acceptable est convenable pour le soin, le conditionnement, le maquillage, l'élimination du maquillage à partir de, la protection, le nettoyage ou le lavage des fibres kératiniques.

10. Utilisation selon au moins l'une des revendications 5 à 9, **caractérisée en ce que** l'agent destiné à modifier la forme de fibres kératiniques se trouve dans une composition qui favorise la pénétration dans le follicule pileux.

11. Utilisation selon au moins l'une des revendications 5 à 10, **caractérisée en ce que** la composition contient également au moins un adjuvant choisi parmi les silicones sous forme soluble, dispersée ou micro-dispersée, les agents tensioactifs non ioniques, anioniques, cationiques et amphotères, les céramides, glyco-céramides et pseudo-céramides, les vitamines et provitamines, y compris le panthénol, les huiles végétales, animales, minérales et synthétiques, les cires autres que les céramides, glyco-céramides et pseudo-céramides, les écrans solaires à base de silicone ou non à base de silicone, hydrosolubles et liposolubles, les agents perlés et les opacifiants, les agents séquestrant, les plastifiants, les agents solubilisants, les agents acidifiants, les épaississants minéraux et organiques, les antioxydants, les acides hydroxylés, les agents de pénétration, les parfums et les conservateurs.

12. Utilisation selon au moins l'une des revendications 5 à 11, **caractérisée en ce que** la composition est une composition cosmétique.
